Europäisches Patentamt

European Patent Office

Office européen des brevets

(19)

(11) Veröffentlichungsnummer : **0 199 848**
**B1**

(12) **EUROPÄISCHE PATENTSCHRIFT**

(45) Veröffentlichungstag der Patentschrift :
28.09.88

(51) Int. Cl.⁴ : **A 61 B   1/00, A 61 M 25/00**

(21) Anmeldenummer : 85115210.8

(22) Anmeldetag : 30.11.85

(54) Vorrichtung zum Einführen eines Endoskops oder eines chirurgischen Instruments in Körperhöhlen mit Mitteln zum Zuführen und Absaugen eines Spülmediums.

(30) Priorität : 09.01.85 DE 3500444

(43) Veröffentlichungstag der Anmeldung :
05.11.86 Patentblatt 86/45

(45) Bekanntmachung des Hinweises auf die Patenterteilung : 28.09.88 Patentblatt 88/39

(84) Benannte Vertragsstaaten :
CH FR GB IT LI NL

(56) Entgegenhaltungen :
DE-A- 2 449 559
DE-A- 2 902 829
DE-B- 2 428 000
DE-B- 2 850 021
FR-A-   738 956
US-A- 4 424 833

(73) Patentinhaber : AESCULAP-WERKE AG vormals Jetter & Scheerer
Möhringer Strasse 125-146
D-7200 Tuttlingen (DE)

(72) Erfinder : Kolditz, Dietmar, Dr.
Libellenweg 5
D-4630 Bochum (DE)
Erfinder : Keller, Hans
Hauptstrasse 63
D-7201 Dürbheim (DE)
Erfinder : Ernst-Kienzle, Karl
Adamsgasse 1
D-7717 Immendingen 2 (DE)

(74) Vertreter : Hoeger, Stellrecht & Partner
Uhlandstrasse 14c
D-7000 Stuttgart 1 (DE)

**Beschreibung**

Die Erfindung betrifft eine Vorrichtung zur Einführung eines Endoskops oder eines chirurgischen Werkzeuges in Körperhöhlen mit einer Schleuse mit einem in die Körperhöhle einschiebbaren zylindrischen Schaft, in den ein zylindrisches Endoskop oder Werkzeug einschiebbar ist, welches zwischen sich und dem zylindrischen Schaft Längskanäle ausbildet, die über durch die Wand des zylindrischen Schaftes hindurchgehende, außerhalb des Körpers liegende Öffnungen in Verbindung mit einer Zufuhr bzw. einer Absaugung für ein Spülmedium stehen.

Um in Körperhöhlen, beispielsweise im Kniegelenk, mit Optiken diagnostizieren oder operieren zu können, müssen bestimmte Voraussetzungen geschaffen werden, die die natürlichen Formen und Abmessungen so erweitern, daß ein notwendiger Überblick und eine größere Bewegungsfreiheit vorliegen. Um dies zu erreichen, ist es bereits bekannt, Organe oder Gelenke mittels gasförmiger oder flüssiger Medien zu erweitern. Diese Erweiterung kann nur mit einem gewissen Überdruck der innenliegenden Medien zum normal vorhandenen Luftdruck erreicht werden. Beim Einführen von Betrachtungs- oder Behandlungsinstrumenten wird dann ein Druckausgleich herbeigeführt, so daß der ursprünglich vorhandene Zustand des Überdruckes nicht gehalten werden kann.

Zum Einführen in Körperhöhlen sind Vorrichtungen bekannt, bei denen das eigentliche Endoskop oder Werkzeug über eine Schleuse in die Körperhöhle eingeführt wird, wobei diese Schleuse als getrenntes Bauteil mit einem zylindrischen Schaft in die Körperhöhle eingesetzt wird und somit nicht nur ein Auswechseln des in die Körperhöhle eingeschobenen Werkzeuges oder Endoskops ermöglicht, sondern auch eine Abdichtung der Körperhöhle gegenüber dem Außenraum. Derartige Instrumente sind beispielsweise beschrieben in der DE-A-2 428 000 oder in der FR-A-738 956.

Ausgehend von diesem Stand der Technik ist es Aufgabe der Erfindung, eine Vorrichtung der eingangs beschriebenen Art so auszubilden, daß einerseits auch bei verschieden tiefem Einschieben des Endoskops oder Werkzeuges das Spülmedium immer im Bereich des freien Endes des Endoskops oder Werkzeuges aus den Längskanälen austritt bzw. in diesem Bereich auch wieder abgesaugt wird und daß andererseits trotzdem eine einfache Herstellung einer solchen Vorrichtung gewährleistet ist.

Diese Aufgabe wird bei einer Vorrichtung der eingangs beschriebenen Art erfindungsgemäß dadurch gelöst, daß der zylindrische Schaft aus einem äußeren Rohr besteht, welches die Öffnungen aufweist und aus einem in dieses eingeschobenen, inneren Rohr, in dessen Wand Langlöcher vorgesehen sind, die mit jeweils einer der zwei Öffnungen des äußeren Rohres ausgerichtet sind, und daß das eingeschobene, zylindrische Endoskop oder Werkzeug mit seiner Außenwand dicht an der Innenwand des inneren Rohres anliegt und somit zusammen mit den Langlöchern abgedichtete Längskanäle für das Spülmedium bildet.

Durch diesen Aufbau ergibt sich eine besonders einfache Herstellung eines auf der Innenseite mit Längsnuten versehenen Schaftes, der im Zusammenhang mit dem zylindrischen Instrument abgeschlossene Längskanäle bildet. Diese Ausgestaltung ermöglicht es dem Operateur auch, das freie Ende des Endoskops oder des Werkzeuges freizuspülen, falls sich hier Gewebeteilchen oder dergleichen angelagert haben. Dies wird dadurch ermöglicht, daß das eingeschobene Instrument selbst die Spülkanäle begrenzt, so daß sich die Spülkanäle zwangsläufig zum vorderen Ende des eingeschobenen Endoskops oder Werkzeuges hin öffnen.

An dem außerhalb des Körpers liegenden Teil des Schaftes kann eine Dichtung angeordnet sein, die den Schaft gegen das Endoskop oder Werkzeug abdichtet. Diese Dichtung sichert zusätzlich die Körperhöhle gegen einen Druckausgleich mit der Umgebung. Dabei ist es vorteilhaft, wenn sich die Dichtung in einem an den Schaft angesetzten Dichtkörper befindet und in ihm mittels eines Überwurfringes gehalten ist.

Bei einem weiteren bevorzugten Ausführungsbeispiel ist vorgesehen, daß der Schaft im Bereich seines freien Endes eine nach außen weisende Verdickung aufweist. Diese hat den Vorteil, daß sie sich nach dem Einschieben der Schleuse fest gegen das umliegende Gewebe andrückt und somit ein unbeabsichtigtes Herausziehen der Schleuse verhindert. Es ist vorteilhaft, wenn die Verdickung auf der dem freien Ende des Schaftes abgewandten Seite der im Körperinnern angeordneten Öffnungen liegt, die mit jeweils einem der beiden Längskanäle in Verbindung stehen. Dann verschließt nämlich die Verdickung die Körperhöhle an der Einstichstelle und verhindert somit zusätzlich einen Druckausgleich zwischen der Körperhöhle und der Umgebung. Außerdem wird verhindert, daß aus den Öffnungen austretendes Spülmedium in die Umgebung entweicht, d. h. dieses Spülmedium wird durch diese Maßnahme gezielt in die Körperhöhle eingeleitet.

Besonders vorteilhaft ist es, wenn die Verdickung kegelstumpfförmig ausgebildet ist und sich zum freien Ende des Schaftes hin verjüngt. Dadurch wird das Einschieben der Schleuse in die Körperhöhle erleichtert, während ein Herausziehen nur durch Aufbringen einer kontrollierten, größeren Kraft möglich ist.

Bei einer weiteren bevorzugten Ausgestaltung ist vorgesehen, daß das Endoskop oder das Werkzeug von einer zylindrischen Schutzhülse umgeben ist. Diese Schutzhülse schützt beispielsweise eine Faseroptik gegen Beschädigung durch Abknicken oder durch in der Umgebung verwendete Instrumente. Vorteilhaft ist es dabei, wenn die Schutzhülse zumindest an der Außenseite elektrisch isolierend ausgebildet ist, da auf diese Weise auch durch eine Elektrokoagulation in der Umgebung des Instrumentes keine Beschädigung des Instrumentes möglich ist.

Die über das Endoskop oder Werkzeug geschobene Schutzhülse kann lösbar mit dem Endoskop oder Werkzeug verbunden sein.

Die nachfolgende Beschreibung einer bevorzugten Ausführungsform der Erfindung dient im Zusammenhang mit der Zeichnung der näheren Erläuterung. Es zeigen :

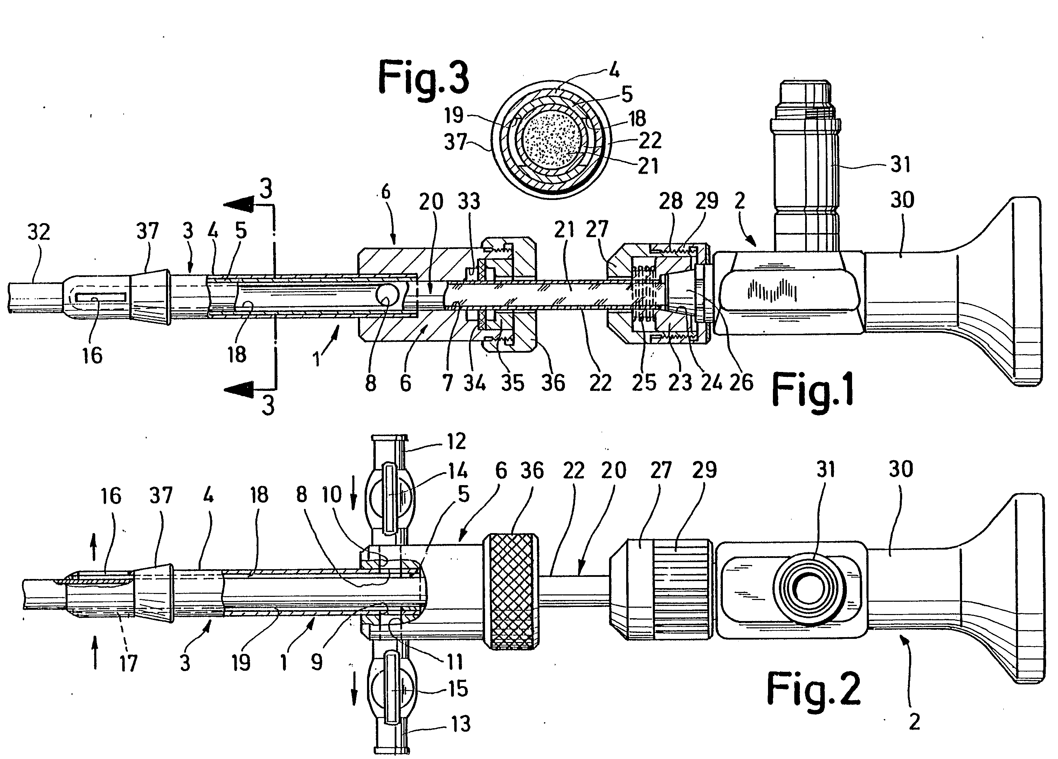

Figur 1 : eine Seitenansicht einer teilweise geschnitten dargestellten Vorrichtung zur Einführung eines Endoskops in den Körper ;

Figur 2 : eine Draufsicht auf die Vorrichtung der Figur 1 und

Figur 3 : eine Schnittansicht längs Linie 3-3 in Figur 1.

Die in der Zeichnung dargestellte Vorrichtung umfaßt zwei Teile, nämlich ein Schleusenteil 1 und ein Instrument 2, im vorliegenden Falle ein Endoskop zur Betrachtung eines Operationsgebietes.

Das Schleusenteil 1 weist einen zylindrischen Schaft 3 auf, der im wesentlichen aus einem äußeren Rohr 4 und einem in dieses eingeschobenen inneren Rohr 5 besteht, welches flächig an der Innenwand des äußeren Rohres anliegt. Beide Rohre 4 und 5 sind mit einem Ende in einen Dichtungskörper 6 eingesetzt und fluchten mit einer Bohrung 7 in dem Dichtungskörpers, dessen Durchmesser der lichten Weite des inneren Rohres 5 entspricht.

Das äußere Rohr 4 weist in seinem in den Dichtungskörper 6 eingesetzten Teil zwei diametral einander gegenüberliegende Öffnungen 8 und 9 auf, die mit radialen Kanälen 10 bzw. 11 im Dichtungskörper 6 in Verbindung stehen. An die Kanäle 10 und 11 schließen sich Anschlüsse 12 bzw. 13 für eine in der Zeichnung nicht dargestellte Zufuhrleitung und eine in der Zeichnung ebenfalls nicht dargestellte Absaugleitung für ein Spülmedium an. In den Anschlüssen 12 und 13 sind Dosierventile angeordnet, die mittels Drehgriffen 14 bzw. 15 in der gewünschten Weise eingestellt werden können.

Am gegenüberliegenden Ende sind in dem äußeren Rohr 4 zwei weitere einander diametral gegenüberliegende Öffnungen 16 und 17 angeordnet, die den Innenraum des äußeren Rohres 4 mit der Umgebung verbinden.

Das innere Rohr 5 weist zwei einander diametral gegenüberliegende Langlöcher 18 bzw. 19 auf, die jeweils mit Öffnungen 8 und 16 bzw. 9 und 17 im äußeren Rohr 4 ausgerichtet sind und somit diese Öffnungen miteinander verbinden.

In das innere Rohr 5 des Schaftes 3 ist ein rohrförmiger Teil 20 des Instrumentes 2 einschiebbar. Es handelt sich dabei im dargestellten Ausführungsbeispiel um eine kreiszylindrische Glasfaseroptik 21, die von einer rohrförmigen Schutzhülse 22 umgeben ist. Diese Schutzhülse kann aus Metall bestehen, es ist auch möglich, die Schutzhülse aus einem sterilisierbaren Kunststoff herzustellen. Bei Verwendung von Metall ist es günstig, wenn die Schutzhülse 22 eine elektrisch isolierende Außenschicht aufweist, beispielsweise eine Oxidschicht oder einen Kunststoffüberzug.

Diese Schutzhülse 22 ist auf die Glasfaseroptik 21 aufgeschoben und wird in seiner Lage an dem Instrument dadurch fixiert, daß ein Endstück 23 mit einer konischen Ausnehmung 24 mittels einer Druckfeder 25 gegen einen die Glasfaseroptik umgebenden konischen Ansatz 26 des Endoskops gedrückt wird. Dazu stützt sich die Druckfeder 25 einmal an dem Endstück und zum anderen an einer Überwurfhaube 27 ab, die in ein Innengewinde 28 einer den Ansatz 26 umgebenden Hülse 29 aufgeschraubt ist. Die Glasfaseroptik, der Ansatz 26 und die Hülse 29 sind mit einem griffförmig ausgebildeten Teil 30 verbunden, an welches über einen radial abstehenden Stutzen 31 ein weiterer, in der Zeichnung nicht dargestellter Lichtleiter angeschlossen werden kann, durch den das über die Glasfaseroptik 21 einfallende Licht einer in der Zeichnung nicht dargestellten, an sich bekannten Betrachtungseinrichtung, beispielsweise einer Fernsehkamera, zugeführt werden kann.

Der Außendurchmesser der Schutzhülse 22 entspricht dem Innendurchmesser des inneren Rohres 5, so daß beim Einschieben des rohrförmigen Teils 20 in den Schleusenteil 1 die Schutzhülse 22 dichtend an der Innenwand des inneren Rohres 5 anliegt und zusammen mit den Langlöchern 18 und 19 sowie der Innenwand des äußeren Rohres 4 zwei voneinander getrennte Kanäle für das zugeführte bzw. das abgesaugte Spülmedium ausbildet.

Das Teil 20 ist dabei länger ausgebildet als der Schaft 3, so daß das freie Ende 32 des Teils 20 aus dem Schaft hervorsteht. Dieses freie Ende ist beispielsweise der Lichteintritt der Glasfaseroptik ; bei Verwendung eines bearbeitenden Werkzeuges anstelle einer Glasfaseroptik kann das freie Ende beispielsweise einen Fräskopf tragen, der durch eine innerhalb der Schutzhülse 22 angeordnete Antriebswelle antreibbar ist.

Im Dichtungskörper 6 ist in einer stufenförmig erweiterten Bohrung 33 eine Dichtscheibe 34 gehalten, die mit ihrem inneren Rand an der Schutzhülse 22 des rohrförmigen Teils 20 anliegt. Die Dichtscheibe 34 wird mittels eines Distanzstückes 35 von einem Überwurfring 36 gegen eine Stufe der Bohrung 33 gedrückt und dadurch in dieser Position gehalten.

Durch die dichte Anlage des rohrförmigen Teils 20 an der Innenwand des inneren Rohres 5 und zusätzlich durch die Dichtscheibe 34 wird das Schleusenteil 1 gegenüber dem eingeschobenen rohrförmigen Teil 20 abgedichtet.

Das äußere Rohr 4 trägt unmittelbar neben den Öffnungen 16 und 17 am freien Ende des Schaftes 3 eine kegelstumpfförmige Verdickung 37, die sich zum freien Ende des Schaftes hin verjüngt, so daß sie das Einschieben des Schleusenteils in den Körper nicht behindert, den Schleusenteil aber nach dem Einschieben im Körper festhält.

Nach dem Einschieben des Schleusenteils in den Körper kann das rohrförmige Teil 20 des Instrumentes 2 in den Schleusenteil eingeschoben werden. Die Zufuhr des Spülmediums und dessen Absaugung erfolgt ausschließlich durch den Schleusenteil, d. h. das Instrument selbst wird dafür nicht benötigt, so daß es ohne jede Behinderung im Schleusenteil verdrehbar oder in Längsrichtung verschiebbar ist. Durch die Abdichtung des Schleusenteils gegenüber dem Instrument kann der erhöhte Innendruck im Körper aufrechterhalten werden, auch wenn die Position des Instrumentes im Schleusenteil verändert wird. Ebenfalls ohne Druckverlust ist es möglich, das Eintrittsfenster eines Endoskops bzw. das Werkzeug eines bearbeitenden Instrumente zu reinigen, in dem man das zylindrische Teil 20 so weit aus dem Schleusenteil herauszieht, daß das optische Fenster bzw. das Werkzeug in den Bereich der Öffnungen 16 und 17 gelangt. Dadurch strömt das Spülmedium unmittelbar am Fenster bzw. am Werkzeug vorbei und reinigt dieses. Schiebt man das Instrument anschließend wieder weiter in den Schleusenteil ein, tritt das Spülmedium wieder in der gewünschten Weise durch die Öffnung 16 in die Umgebung aus und wird durch die Öffnung 17 wieder angesaugt.

**Patentansprüche**

1. Vorrichtung zur Einführung eines Endoskops oder eines chirurgischen Werkzeuges in Körperhöhlen mit einer Schleuse (1) mit einem in die Körperhöhle einschiebbaren zylindrischen Schaft (3), in den ein zylindrisches Endoskop oder Werkzeug (20) einschiebbar ist, welches zwischen sich und dem zylindrischen Schaft (3) Längskanäle ausbildet, die über durch die Wand des zylindrischen Schaftes (3) hindurchgehende, außerhalb der Körperhöhle liegende Öffnungen (8, 9) in Verbindung mit einer Zufuhr bzw. einer Absaugung (12, 13) für ein Spülmedium stehen, dadurch gekennzeichnet, daß der zylindrische Schaft (3) aus einem äußeren Rohr (4) besteht, welches die Öffnungen (8, 9) aufweist, und aus einem in dieses eingeschobenen, inneren Rohr (5) in dessen Wand Langlöcher (18, 19) vorgesehen sind, die mit jeweils einer der beiden Öffnungen (8 bzw. 9) des äußeren Rohres (4) ausgerichtet sind, und daß das eingeschobene, zylindrische Endoskop oder Werkzeug (20) mit seiner Außenwand dicht an der Innenwand des inneren Rohres (5) anliegt und somit zusammen mit den Langlöchern (18, 19) die abgedichteten Längskanäle für das Spülmedium bildet.

2. Vorrichtung nach Anspruch 1, dadurch gekennzeichnet, daß an dem außerhalb des Körpers liegenden Teil des Schaftes (3) eine Dichtung (34) angeordnet ist, die den Schaft (3) gegen das Endoskop oder das Werkzeug (20) abdichtet.

3. Vorrichtung nach Anspruch 2, dadurch gekennzeichnet, daß sich die Dichtung (34) in einem an den Schaft (3) angesetzten Dichtungskörper (6) befindet und in ihm mittels eines Überwurfringes (36) gehalten ist.

4. Vorrichtung nach einem der voranstehenden Ansprüche, dadurch gekennzeichnet, daß der Schaft (3) im Bereich seines freien Endes eine nach außen weisende Verdickung (37) aufweist.

5. Vorrichtung nach Anspruch 4, dadurch gekennzeichnet, daß die Verdickung (37) auf der vom freien Ende des Schaftes (3) abgewandten Seite von im Körperinneren angeordneten Öffnungen (16, 17) liegt, die mit jeweils einem der beiden Längskanäle in Verbindung stehen.

6. Vorrichtung nach Anspruch 4 oder 5, dadurch gekennzeichnet, daß die Verdickung (37) kegelstumpfförmig ausgebildet ist und sich zum freien Ende des Schaftes (3) hin verjüngt.

7. Vorrichtung nach einem der voranstehenden Ansprüche, dadurch gekennzeichnet, daß das Endoskop oder Werkzeug von einer zylindrischen Schutzhülse (22) umgeben ist.

8. Vorrichtung nach Anspruch 7, dadurch gekennzeichnet, daß die Schutzhülse (22) zumindest an der Außenseite elektrisch isolierend ausgebildet ist.

9. Vorrichtung nach einem der Ansprüche 7 oder 8, dadurch gekennzeichnet, daß die über das Endoskop oder Werkzeug geschobene Schutzhülse (22) lösbar mit dem Endoskop oder Werkzeug verbunden ist.

**Claims**

1. A device for inserting an endoscope or a surgical instrument into body cavities with a sluice (1) having a cylindrical shaft (3) insertable into the body cavity and into which a cylindrical endoscope or instrument (20) may be inserted, the endoscope or instrument forming longitudinal channels between itself and the cylindrical shaft (3), the longitudinal channels being connected to a feed or suction pipe (12, 13) for a flushing medium via apertures (8, 9) lying outside the body cavity and penetrating the wall of the cylindrical shaft (3), characterised in that the cylindrical shaft (3) consists of an outer tube (4) having apertures (8, 9) and of an inner tube (5) inserted into the outer tube (4), the wall of the inner tube (5) having longitudinal holes (18, 19) each aligned with a respective one of the two apertures (8, 9) of the outer tube (4), and in that the outer wall of the inserted cylindrical endoscope or instrument (20) is flush with the inner wall of the inner tube (5) and thus, together with the longitudinal holes (18, 19), forms the sealed longitudinal channels for the flushing medium.

2. A device according to claim 1, characterised in that a seal (34) is disposed on the portion of the shaft (3) lying outside the body for sealing the shaft (3) with respect to the endoscope or the instrument (20).

3. A device according to claim 2, characterised in that the seal (34) is located in a seal body (6) disposed on the shaft (3) and is held therein by means of a coupling ring (36).

4. A device according to one of the preceding claims, characterised in that the shaft (3) has an outwardly oriented thickening (37) at its free end.

5. A device according to claim 4, characterised in that the thickening (37) lies on the side, which faces away from the free end of the shaft, of apertures (16, 17) disposed in the body interior and each connected to a respective one of the two longitudinal channels.

6. A device according to claim 4 or 5, characterised in that the thickening (37) is frustoconical and tapers inwardly towards the free end of the shaft (3).

7. A device according to one of the preceding claims, characterised in that the endoscope or instrument is surrounded by a cylindrical protective sleeve (22).

8. A device according to claim 7, characterised in that the protective sleeve (22) is formed so as to be electrically insulating at least on the outside.

9. A device according to one of claims 7 or 8, characterised in that the protective sleeve (22) pushed over the endoscope or instrument is detachably connected to the endoscope or instrument.

**Revendications**

1. Dispositif pour introduire un endoscope ou un outil chirurgical dans une cavité corporelle, équipé d'une traversée étanche (1) qui comprend elle-même une tige cylindrique (3) destinée à être enfoncée dans la cavité corporelle et dans laquelle on peut enfoncer un endoscope ou outil cylindrique (20) qui forme entre lui-même et la tige cylindrique (3) des canaux longitudinaux qui sont en communication avec une arrivée et une aspiration (12, 13) de fluide d'irrigation, par des ouvertures (8, 9) qui traversent la paroi de la tige cylindrique (3) et qui sont situées à l'extérieur de la cavité du corps, respectivement, caractérisé en ce que la tige cylindrique (3) est composée d'un tube extérieur (4) qui présente les ouvertures (8, 9) et d'un tube intérieur (5) emmanché dans le premier et dans la paroi duquel sont prévus des trous allongés (18, 19) qui sont alignés respectivement sur les deux ouvertures (8, 9) du tube extérieur (4), et en ce que l'endoscope ou outil cylindrique enfoncé (20) est en appui à joint étanche par sa surface externe contre la surface interne du tube intérieur (5) et forme, en combinaison avec les trous allongés (18, 19), les canaux longitudinaux étanches de circulation du fluide d'irrigation.

2. Dispositif selon la revendication 1, caractérisé en ce que, sur la partie de la tige (3) située à l'extérieur du corps du patient, est disposée une garniture d'étanchéité (34) qui ferme le joint entre la tige (3) et l'endoscope ou l'outil (20).

3. Dispositif selon la revendication 2, caractérisé en ce que la garniture d'étanchéité (34) est logée dans un bloc d'étanchéité (6) monté sur la tige (3) et est retenue dans ce bloc au moyen d'une virole chapeau (36).

4. Dispositif selon une des revendications précédentes, caractérisé en ce que la tige (3) présente un renflement (37) en saillie vers l'extérieur dans la région de son extrémité libre.

5. Dispositif selon la revendication 4, caractérisé en ce que le renflement (37) se trouve à l'opposé de l'extrémité libre de la tige (3) par rapport aux ouvertures (16, 17) situées à l'intérieur du corps du patient et qui sont respectivement en communication avec les deux canaux longitudinaux.

6. Dispositif selon la revendication 4 ou la revendication 5, caractérisé en ce que le renflement (37) est de forme tronconique et se rétrécit vers l'extrémité libre de la tige (3).

7. Dispositif selon une des revendications précédentes, caractérisé en ce que l'endoscope ou l'outil est entouré d'un manchon de protection cylindrique (22).

8. Dispositif selon la revendication 7, caractérisé en ce que le manchon de protection (22) est isolant de l'électricité, du moins sur sa surface externe.

9. Dispositif selon une des revendications 7 et 8, caractérisé en ce que le manchon de protection (22) emmanché sur l'endoscope ou sur l'outil est fixé à l'endoscope ou à l'outil par une liaison démontable.

Fig.1

Fig.2

Fig.3